# EUROPEAN PATENT SPECIFICATION

(11) **EP 3 764 876 B1**
(45) Date of publication and mention of the grant of the patent: **11.06.2025**
(21) Application number: 19723535.1
(22) Date of filing: 08.04.2019
(51) Int. Cl.: A61B 5/00, A44C 5/00, A61B 10/00, A61K 49/00

(54) **APPARATUS FOR ASSESSING SKIN REACTIVITY TO AN IMPLANT MATERIAL**
VORRICHTUNG ZUR BEURTEILUNG DER HAUTREAKTIVITÄT AUF EIN IMPLANTATMATERIAL
APPAREIL D'ÉVALUATION DE LA RÉACTIVITÉ DE LA PEAU À UN MATÉRIAU D'IMPLANT

(30) Priority: 09.04.2018 US 201815948620
(43) Date of publication of application: 20.01.2021
(73) Proprietor: Drzala, Mark R., Morristown, NJ 07960 (US); Reiter, Mitchell F., Bernardsville, NJ 07924 (US)
(72) Inventor: Drzala, Mark R., Morristown, NJ 07960 (US); Reiter, Mitchell F., Bernardsville, NJ 07924 (US)
(74) Representative: Meissner Bolte Nürnberg
(86) International application number: PCT/US2019/026306
(87) International publication number: WO 2019/199660

(56) References cited:
- FR-A1- 3 040 868
- US-A- 4 904 448
- US-A1- 2011 306 898
- US-A1- 2016 299 093

## Description

### CROSS-REFERENCE TO RELATED APPLICATIONS

This application claims priority under 35 U.S.C. §119(e) to United States Patent Application No. 15/948,620, filed April 9, 2018.

### FIELD OF THE INVENTION

The present invention relates generally to an apparatus for assessing skin reactivity to a test material and more specifically relates to an apparatus for assessing skin reactivity to medical implant materials.

### BACKGROUND OF THE INVENTION

Surgical procedures involving metallic implants are increasingly common. In the United States alone, more than 1 million lower extremity total joint replacements are performed each year. In addition, three million Americans have dental implants, and that number has been increasing by 500,000 per year. Some of these patients go on to experience adverse events related to their implants due to hypersensitivity to the implanted metal, such as hip and knee replacements. Determining if someone is hypersensitive to specific implant materials would provide very useful information to patients who are considering implant surgery or who may already have undergone such a procedure.

Presently, persons who suspect that they are sensitive to metals are typically referred for skin patch-testing under the care of a dermatologist for confirmation. Another less common option available to test for hypersensitivity to metals is a blood test called a lymphocyte transformation test. Both types of testing are typically ordered by a physician (typically an allergist), are time-consuming, costly, and are not performed routinely. Additionally, it remains unknown which patients need to undergo evaluation, and there is no defined standard for determining what constitutes a positive test for potential hypersensitivity to various metals. There are currently no over-the-counter kits or devices that allow patients to undergo some form of preliminary assessment to test for cutaneous hypersensitivity to topically applied metals.

It is important to note that many patients who prove to be hypersensitive to the metallic implants do not have a history of previous hypersensitivity to metal jewelry. This suggests that potentially all patients could benefit from metallic hypersensitivity testing and this is currently only available through costly formal dermatologist evaluation and follow up.

FR 3 040 868 A1 discloses a device for detecting a risk of allergic skin reaction to at least one component of an implant intended to be implanted in a patient. The detection device comprises a plurality of detection pellets made of different allergenic materials and configured to be arranged on the skin of a patient.

US 2011/306898 A1 discloses a patch for IBS related testing that is attachable to skin and comprises one or more test materials, with the patch constructed such that at least one test material, selected from the group consisting of substances that can cause allergic contact dermatitis, may be brought into contact with skin to which the patch may be attached.

US 4 904 448 A discloses a patch test material for the detection of metal allergies which contains a dental metal, noble or base, with which an allergic reaction aid such as a biological high molecular weight compound, e.g., a protein is present.

Thus, there remains a strong need for a non-invasive, reliable, convenient, and inexpensive method for detecting the presence of skin reactivity to materials, such as medical implant materials.

### SUMMARY OF THE INVENTION

The invention is set forth in the independent claims. Embodiments result from the dependent claims and the below description.

This disclosure addresses the need mentioned above in a number of aspects. In one aspect, this disclosure provides an apparatus for assessing skin reactivity to a test material, *e.g.*, an implant material. The apparatus includes a holder and a test material unit. The holder further comprises a test portion and a holding portion. The holder can be formed of an inert and hypoallergenic material. The test portion comprises a concaved housing compartment with an opening facing towards the skin of a subject when the apparatus is worn. The concaved housing compartment is adapted to receive and contact the test material unit therein. The periphery of the test material unit matches the shape of an inner surface of the concaved housing compartment. The holding portion is configured to cause the test material unit to directly contact with the skin, without using an adhesive. The holding portion is one of a bracelet, a band (*e.g.*, elastic band), a wristband, a pendant, a chest band, a leg band, a necklace, an an anklet, a belt, a waist belt, a ring, an earring, and a watch-band style holder. The test material unit comprises an implant material and is mountable to the concaved housing compartment of the holder.

In some embodiments, the test material unit can be removably mounted to the concaved compartment using a snap-fit mechanism.

In some embodiments, the implant material comprises an orthopedic surgery metal, a reconstructive surgery metal, or an alloy of implant metals, or implant metal of different metallic combinations.

In some embodiments, the implant material comprises an implant metal, such as chromium, cobalt, molybdenum, titanium, vanadium, aluminum, palladium, zirconium, yttrium, tin, tantalum, niobium, copper, gold, silver, manganese, tungsten, beryllium, or nickel, or an alloy or combination thereof.

In some embodiments, the implant material comprises an implant metal alloy containing any one of aluminum, chromium, cobalt, molybdenum, nickel, vanadium, titanium, and Ti6AI-4V. In some embodiments, the orthopedic surgery metal is a metal alloy of chromium, cobalt, or molybdenum. In some embodiments, the orthopedic surgery metal may also include a titanium alloy (*e.g.*, Ti6Al-4V has 90% titanium, 6 % aluminum, and 4% vanadium, 0.25% iron and 0.2% oxygen).

In some embodiments, the implant material comprises a non-metallic reconstructive surgery material, or a non-metallic implant, or a non-metallic orthopedic surgery material selected from the group consisting of polymethyl methacrylate (PMMA), polyethylene, polyaryletherketone (PEEK), and carbon fiber.

In some embodiments, the implant material comprises a non-metallic surgery material selected from the group consisting latex, styrax, balsam of Peru, gum mastic (in Mastisol), and Gutta-Percha.

In some embodiments, the implant material comprises a dental implant material, such as titanium, zirconium, aluminum, vanadium, a dental amalgam consisting essentially of liquid mercury, or a metal alloy mixture having mercury, silver, tin, copper, or combinations of two or more thereof.

In some embodiments, the implant material comprises a first metal implant material and a second metal implant material that are jointly attached against each other, such that only the first metal implant material or the second metal implant material directly contacts the skin. The first metal implant material and the second metal implant material can be different materials. In some embodiments, the first metal implant material and the second metal implant material are removably attached against each other or fabricated as one piece.

The test material unit comprises a top surface and a bottom surface. In some embodiments, the top surface is smaller than the bottom surface and mounted in the concaved housing compartment of the holder.

In some embodiments, the shape of the inner surface of the concaved housing compartment can be circular, elliptical, square, rectangular, polygonal, quadrilateral, square, triangular, parallelogram, pentagonal, hexagonal, heptagonal or octagonal.

In some embodiments, the holding portion of the holder further comprises a fastening module, such as a hook-and-loop, a button closure, and a buckle closure.

In some embodiments, the test material unit comprises an identification embossed on a surface thereof. The identification may include a test material type, a manufacture date, a serial code, a medical provider name, or a combination of two or more thereof.

In another aspect, this disclosure also provides a method for assessing skin reactivity of a subject to a test material. The method is performed prior to the subject undergoing implant surgery, and an implant material is used as the test material to test tissue sensitivity of the subject to the implant material being implanted. The method may including: (i) applying the apparatus, as described above, on the skin of the subject, such that the test material unit of the apparatus makes direct contact with the skin for a predetermined period of time; and (ii) determining a presence of skin reactivity based on one or more cutaneous reactions of the skin with direct contact with the test material unit of the apparatus.

In another aspect of the disclosure, a kit for assessing skin reactivity to a test material is also presented. The kit includes one or more holders, one or more test material units described above, and optionally instructions for using the kit.

The foregoing summary is not intended to define every aspect of the disclosure, and additional aspects are described in other sections, such as the following detailed description. The entire document is intended to be related as a unified disclosure. Other features and advantages of the disclosure will become apparent from the following detailed description. It should be understood, however, that the detailed description and the specific examples, while indicating specific embodiments of the disclosure, are given by way of illustration only, because various changes and modifications within the scope of the disclosure will become apparent to those skilled in the art from this detailed description.

### BRIEF DESCRIPTION OF THE DRAWINGS

FIG. 1 shows an example of an apparatus for assessing skin reactivity to a test material which contains a wristband-style holder.
FIG. 2 shows an example of a test material unit mountable to the holder as illustrated in FIG. 1.
FIG. 3 shows an example of an apparatus with a wristband-style holder worn around the wrist of a subject.

### DETAILED DESCRIPTION OF THE INVENTION

The advent of orthopedic and other medical implants has transformed the treatment of bone and joint disorders as well as other medical conditions by improving quality of life, increasing mobility, and reducing pain in affected patients. Most implants used in reconstructive surgical procedures are composed of metal alloys that contain classic contact allergens such as nickel, cobalt, and chromium. Extensive medical literature now exists describing patients experiencing hypersensitivity reactions to implant materials following their surgical procedures. Potential allergic complications after implantation of orthopedic metal devices include cutaneous eruptions, chronic joint pain, edema, implant loosening, and joint failure. In some cases, the allergic complications have led to the need for additional surgery to remove the implant containing the offending material.

Despite the fact that allergies are on the rise in the western world, testing patients prior to surgical procedures involving implants is not routinely performed. The current testing techniques are not easy to interpret and typically involve referral to a dermatologist for skin testing. It has been proposed that previous cutaneous reaction to worn metals is an excellent predictor of metallic hypersensitivity. Since many reconstructive surgeries can be performed using implants of different metallic compositions, knowing about a patient's hypersensitivities can allow for modification of the procedure to avoid implants that contain the specific metals to which the patient is hypersensitive.

To address this need, the present disclosure provides an apparatus for assessing skin reactivity to a test material, *e.g.*, an implant material. The disclosed apparatus can be adapted to meet the specific needs of dentists, surgeons, and/or other physicians to help provide information that could assist in determining which patients may require referral to specialists (*i.e.*, allergists) for more extensive testing. The information gained may also help dentists and surgeons determine more appropriate treatments and may assist them in choosing safer implants, for example, avoiding the use of dental amalgam and certain metals in potentially hypersensitive individuals.

The apparatus includes a holder and a test material unit. The holder is configured to be worn on a body part of a wearer, such as ankles, fingers, neck, etc. The holder further includes a test portion and a holding portion. The test portion includes a concave housing compartment with an opening facing towards the skin of a subject when the apparatus is worn. The concaved housing compartment is adapted to receive and contact the test material unit therein. The periphery of the test material unit substantially matches the shape of the inner surface of the concave housing compartment. The holding portion is configured to cause the test material unit to have direct contact with the skin of the subject. The test material unit includes a test material and is removably mounted to the concave housing compartment of the test portion of the holder.

The disclosed apparatus has several advantages. The apparatus can be fabricated into something that will appear innocuous in public. It is important to note that in order to obtain reliable skin reactivity information, surgeons need patients to wear a product for an extended period of time, which is unsuitable for adhesive strips. It is typical that a product must be worn by a patient for a week to ten days. Also, surgeons want the product to be worn the entire time except for bathing and unless a significant adverse reaction occurs. The disclosed apparatus with a durable test material allows patients to conveniently take off and put on the apparatus when needed (for example, when taking a bath), an advantage absent from adhesive strips.

The term "implant," as used herein, refers to a medical device manufactured to replace a missing biological structure, support a damaged biological structure, or enhance an existing biological structure. Medical implants are man-made devices, in contrast to a transplant, which is a transplanted biomedical tissue. The surface of implants that contact the body might be made of a biomedical material, such as titanium, silicone, or apatite depending on what is the most functional. In some cases, implants, contain electronics, *e.g.*, artificial pacemaker and cochlear implants. Some implants are bioactive, such as subcutaneous drug delivery devices in the form of implantable pills or drug-eluting.

The term "substantially," as used herein, refers to the complete or nearly complete extent or degree of an action, characteristic, property, state, structure, item, or result. For example, an object that is "substantially match" would mean that the object is either completely match or nearly completely match.

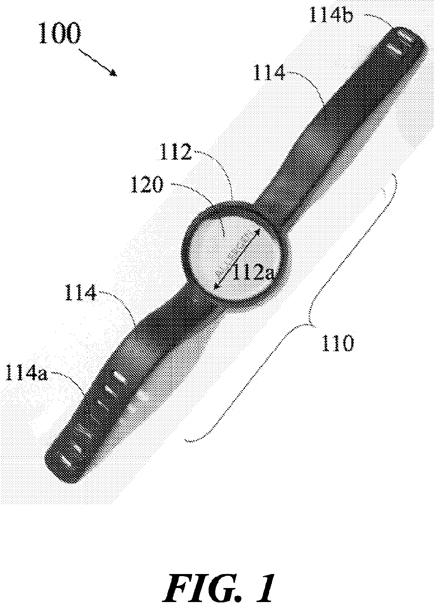

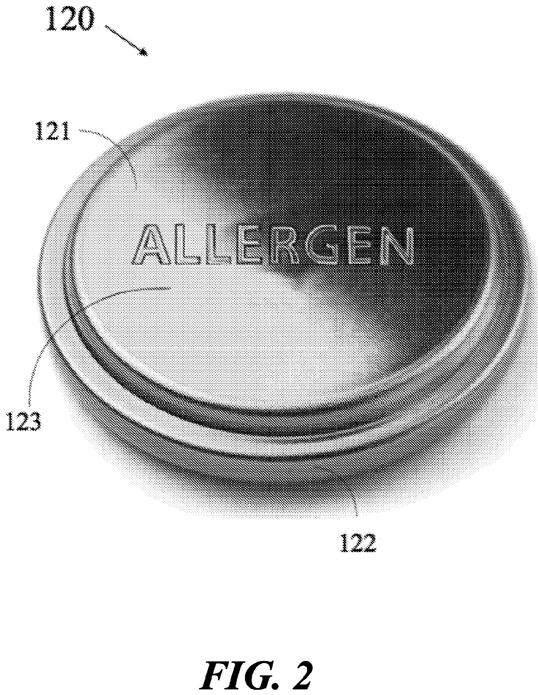

The shape of the inner surface of the concave housing compartment of the holder may include, without limitation, circular, elliptical, square, rectangular, and polygonal, quadrilateral, square, triangular, parallelogram, pentagonal, hexagonal, heptagonal and octagonal. For example, the holder illustrated in **FIG. 1** contains a concave housing compartment having a circular inner surface. Likewise, the shape of the test material unit may include, without limitation, circular, elliptical, square, rectangular, and polygonal, quadrilateral, square, triangular, parallelogram, pentagonal, hexagonal, heptagonal and octagonal, such that the shape of the inner surface of the concave housing compartment of the holder substantially matches that of the test material unit. For example, the test material units illustrated in **FIG. 1** and **FIG. 2** have a circular shape.

The holder can be a wearable bracelet or any other wearable articles. Examples of wearable articles include a bracelet, a band, a wristband, a pendant, a chest band, a leg band, a necklace, an anklet, a belt, a waist belt, a ring, an earring, and a watch-band style holder. For example, a band for testing an implant material can be adapted to be worn on various body parts (*e.g.*, wrists, ankles, elbows, knees, neck, fingers, and toes).

The holding portion of the holder can be an elastic band. The holding portion of the holder can further include a fastening means. In some embodiments, the holding portion of the holder further is adjustable or stretchable, allowing for the material being tested to be firmly held and secured against the subject's skin.

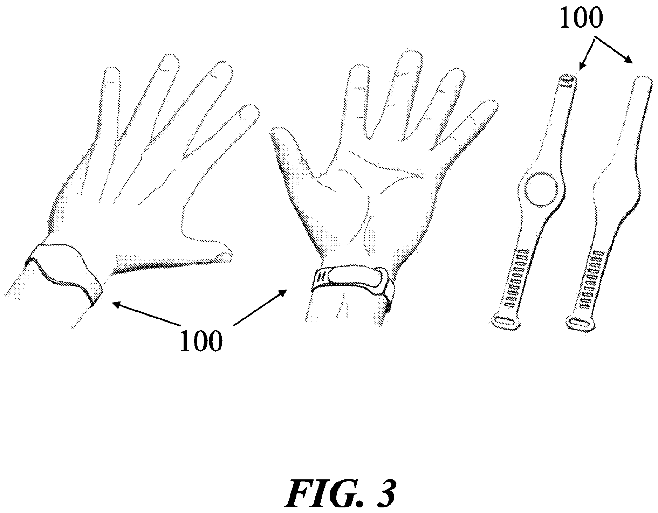

Referring to **FIG. 1****,** an exemplary apparatus **100** for assessing skin reactivity to a test material is illustrated. The apparatus **100** contains a wristband-style holder **110** and a test material unit **120.** The holder **110** further includes a test portion **112** and a holding portion **114.** The test portion **112** includes a concave housing compartment **112a** adapted to receive and contact the test material unit **120** therein. The periphery of the test material unit **120** substantially matches the shape of the inner surface of the concave housing compartment **112a.** The holding portion **114** is configured to cause the test material unit **120** to have direct contact with the skin of the subject. The test material unit **120** includes a test material and is removably mounted to the concave housing compartment **112a** of the test portion **112** of the holder **110.** The holding portion **114** of the holder **110** may further include one or more fastening means **114a** and **114b.** The fastening means **114a** and **114b** of the holding portion **114** can be a hook-and-loop or a buckle closure or a post-hole structure. For example, the fastening means **114a** may include one or more holes, and the fastening means **114b** may include one or more molded posts. The free strap ends of the holding portion **114** of the holder **110** can be adjustably secured together by mating the one or more holes of the fastening means **114a** and the one or more molded posts of the fastening means **114b.** An exemplary apparatus worn by a subject is shown in **FIG. 3****.**

In an embodiment, the holder is made of a hypoallergenic, inert, or latex-free material (*i.e.,* silicone), suitable for holding specific metals or other commonly used medical implant materials in direct contact with the subject's skin. The material to be tested can be worn using the holder in direct contact with the skin for several days. A cutaneous reaction to the applied material could suggest potential hypersensitivity.

Cutaneous skin reactions induced by exposure to the implant material may include, but would not be limited to, the following: red rash, swelling, erythema, eczema, pruritis, burning, urticaria (hives), painful angioedema, blisters, and wheals (welts). These reactions may not appear until 24-72 hours after exposure to the allergen. Those patients eliciting positive skin reactions would be advised to seek out further formal medical evaluation and care with their surgeon, primary care physician, or an appropriate medical specialist (likely a dermatologist or allergist). This information could help direct dentists and surgeons to customize treatment to avoid implants and medical materials that could cause hypersensitivity and lead to complications and adverse sequelae.

The test material unit can be fitted and secured in the concave housing compartment of the test portion of the holder by one or more fastening means. In one example, the concave housing compartment may have a slightly smaller size than the test material unit, such that the test material unit can be fitted and secured in the concave housing compartment with the help of elasticity of the material constituting the holder. In another example, the test material unit can be secured in the concave housing compartment by using a snap-fit mechanism. In yet another example, the inner surface of the concave housing compartment may include an internal thread, and the test material unit may include an external thread. The external thread of the test material unit is configured to be received by the internal thread of the concave housing compartment, whereby the test material unit is rotationally fitted and secured in the concave housing compartment. Alternatively and/or additionally, the test material unit can be secured by one or more fasteners (*e.g.,* screws) or adhesive (*e.g.,* glue). In some embodiments, the holder and the test material unit may be fabricated as a single piece.

The test material unit can include any test materials subjected to a skin hypersensitivity test. The test material may include one or more implant materials commonly used in medicine and dentistry or implant materials that are specific to the needs of certain patient populations. The implant material can be a metallic or non-metallic implant material.

In some embodiments, the implant material can be an implant metal or metal alloy. For example, the implant material can be an orthopedic surgery metal, a reconstructive surgery metal, or an alloy of implant metals, or implant metal of different metallic combinations.

Non-limiting examples of the implant metal may include nickel metal, nickel metal electroplated with nickel sulfate, cobalt metal, cobalt metal electroplated with cobalt chloride, chromium, molybdenum, titanium, amalgam, vanadium, palladium, zirconium, yttrium, tin, tantalum, niobium, copper, gold, silver, aluminum, manganese, tungsten, and beryllium.

Non-limiting examples of the implant metal alloy may include a metal alloy formed of a combination of two or more of nickel, cobalt, chromium, molybdenum, titanium, amalgam, vanadium, palladium, zirconium, yttrium, tin, tantalum, niobium, copper, gold, silver, aluminum, manganese, tungsten, and beryllium. In some embodiments, the implant metal alloy is formed of a combination of two or more of chromium, cobalt, and molybdenum. In one example, the implant metal alloy is a titanium alloy (*e.g.*, Ti6Al-4V has 90% titanium, 6 % aluminum, and 4% vanadium, 0.25% iron and 0.2% oxygen).

The implant material that can be used in the disclosed apparatus may include a wide variety of the specialty implant materials (*e.g.*, metallic or non-metallic implant materials). For example, cardiologists and thoracic surgeons can use the disclosed apparatus for perioperative assessment for metal allergies in the usage of metal sternotomy wire. Neurosurgeons can use the disclosed apparatus for testing metal implants in the spine, titanium implants in the cranium, disc arthroplasty in the cervical and lumbar spine. Cranio-Maxillofacial surgeons can use the disclosed apparatus for testing CMT implants made from metals and metal alloy.

The implant materials can include implant metals commonly used in orthopedic fracture fixation and joint replacement procedures including, without limitation, aluminum, chromium, cobalt, molybdenum, nickel, and titanium, or other identified implant materials.

In another example, the test materials can include non-metallic materials used in orthopedic surgery including, without limitation, polymethyl methacrylate (PMMA or bone cement), polyethylene (joint replacement liners), polyaryletherketone (*e.g.,* PEEK), and carbon fiber.

In yet another example, the test materials can include a dental amalgam-a liquid mercury and metal alloy mixture including, without limitation, mercury, silver, tin, copper, and other trace metals, and combinations of two or more thereof. Dental implants may also include, without limitation, alloys, combinations of metals and pure samples of titanium, zirconium, aluminum, and vanadium. Non-limiting examples of other test materials include palladium, manganese, iron, latex, styrax (found in medical adhesives Mastisol and Tincture of Benzoin), balsam of Peru, gum mastic (in Mastisol), and gutta-percha (implanted in teeth after root canals).

In some embodiments, the test material unit includes an identification on a surface of the test material unit. The identification indicates a test material type, a manufacture date, a serial code, a medical provider name, or a combination of two or more thereof. The identification can also use different colors or shapes to distinguish the material being tested from each other. In some embodiments, the identification is embossed on the surface of the test material unit.

Referring to **FIG. 2****,** an example of a test material unit **120** is illustrated. The test material unit **120** includes nickel a test material. The test material unit contains a top surface **121** and a bottom surface **122.** The top surface **121** may have a different or the same size as compared to the bottom surface **122.** In one example, the top surface **121** may have a smaller size than the bottom surface **122,** as shown in **FIG. 2****.** The smaller top surface **121** is removably received by the housing compartment **112a** of the holder **110** (see **FIG. 1**), and the bottom surface **122** makes direct contact with the skin of a wearer. The top surface **121** can further include an identification **123** (*e.g.,* "ALLERGEN" mark) embossed thereon indicating a test material type (*e.g.*, implant alloy), a manufacture date, a serial code, a medical provider name, or a combination of two or more thereof.

In some embodiments, the test material unit comprises a first implant material (*e.g.,* metal implant material) and a second implant material (*e.g.,* metal implant material). The first implant material and the second implant material being jointly attached against each other, such that the first implant material and the second implant material respectively occupy the opposing portions of the test material unit and only the first implant material or the second implant material makes direct contact with the skin of the subject. In some embodiments, the first implant material and the second implant material are different materials. In some embodiments, the first implant material and the second implant material are removably attached to each other, such that one or more combinations of implant materials are assembled for a single implant material unit.

As used herein, the term "jointly" refers to the proximity of two structures or elements. Particularly, elements that are identified as being "jointly attached" may be either abutting or connected. Such elements may also be near or close to each other without necessarily contacting each other. The exact degree of proximity may in some cases depend on the specific context.

In another aspect, this disclosure also provides a method for assessing skin reactivity of a subject to a test material. The method is performed prior to the subject undergoing implant surgery, and an implant material is used as the test material to test tissue sensitivity of the subject to the implant material being implanted. The method may including (i) applying the apparatus as disclosed on the skin of the subject, such that the test material unit of the apparatus makes direct contact with the skin for a predetermined period of time (*e.g.,* 1 day, 2 days, 3 days, 5 days, 1 weeks, 2 weeks, 3 weeks, 4 weeks, 5 weeks, 6 weeks, 7 weeks, 8 weeks); and (ii) determining a presence of skin reactivity based on one or more cutaneous reactions of the skin with direct contact with the test material unit of the apparatus.

In another aspect of the disclosure, a kit for assessing skin reactivity to a material is also presented. The kit may include one or more holders and one or more test material units described above. The kit may also include instructions for use. For example, the instructions may inform the user of the proper procedure to use the disclosed apparatus on a subject.

A widely available and economical implant material skin hypersensitivity testing kit could allow for early screening of patients scheduled to undergo implant surgeries. The result of this skin sensitivity testing could be used to help provide interested individuals with preliminary information with regards to potential metal hypersensitivities. These individuals could then be directed to medical specialists who could then provide a more definitive diagnosis. It would be left to the treating physician to determine which patients will then need a formal referral to an allergist. The disclose apparatus could provide important preliminary information at a reasonable cost that may assist in the determination as to which patients require more focused testing.

### DEFINITIONS

To aid in understanding the detailed description of the compositions and methods according to the disclosure, a few express definitions are provided to facilitate an unambiguous disclosure of the various aspects of the disclosure. Unless otherwise defined, all technical and scientific terms used herein have the same meaning as commonly understood by one of ordinary skill in the art to which this disclosure belongs.

It is noted here that, as used in this specification and the appended claims, the singular forms "a," "an," and "the" include plural reference unless the context clearly dictates otherwise. The terms "including," "comprising," "containing," or "having" and variations thereof are meant to encompass the items listed thereafter and equivalents thereof as well as additional subject matter unless otherwise noted.

The phrases "in one embodiment," "in various embodiments," "in some embodiments," and the like are used repeatedly. Such phrases do not necessarily refer to the same embodiment, but they may unless the context dictates otherwise.

The terms "and/or" or "/" means any one of the items, any combination of the items, or all of the items with which this term is associated.

As used herein, the term "each," when used in reference to a collection of items, is intended to identify an individual item in the collection but does not necessarily refer to every item in the collection. Exceptions can occur if explicit disclosure or context clearly dictates otherwise.

The use of any and all examples, or exemplary language (*e.g.*, "such as") provided herein, is intended merely to better illuminate the invention and does not pose a limitation on the scope of the invention unless otherwise claimed. No language in the specification should be construed as indicating any non-claimed element as essential to the practice of the invention.

All methods described herein are performed in any suitable order unless otherwise indicated herein or otherwise clearly contradicted by context. In regard to any of the methods provided, the steps of the method may occur simultaneously or sequentially. When the steps of the method occur sequentially, the steps may occur in any order, unless noted otherwise.

Publications disclosed herein are provided solely for their disclosure prior to the filing date of the present invention. Nothing herein is to be construed as an admission that the present invention is not entitled to antedate such publication by virtue of prior invention. Further, the dates of publication provided may be different from the actual publication dates which may need to be independently confirmed.

It is understood that the examples and embodiments described herein are for illustrative purposes only and that the invention is defined by the appended claims.

## Claims

1. An apparatus (100) for contacting a test material with a skin of a subject, comprising:
a holder (110) and
a test material unit (120),
wherein the holder (110) further comprises a test portion (112) and a holding portion (114),
wherein the holder (110) is formed of an inert and hypoallergenic material;
wherein the test portion (112) comprises a concave housing compartment (112a) with an opening facing towards the skin of the subject when the apparatus is worn,
wherein the holding portion is configured to cause a bottom surface (122) of the test material unit (120) to have direct contact with a skin of a subject;
wherein the test material unit (120) consists of an implant metal or metal alloy, wherein the concave housing compartment (112a) is adapted to receive a top surface (121) of the test material unit (120) and
wherein the holding portion (114) of the holder (110) is selected from the group consisting of a bracelet, a band, a wristband, a chest band, a leg band, an anklet, a belt, a waist belt, and a watch-band style holder,
**characterized in that**
the test portion (112) further comprises a closed end facing away from the skin,
the concave housing compartment (112a) is adapted to receive and contact the test material unit (120) therein,
the test material unit (120) is mountable to the concave housing compartment (112a) of the test portion (112) of the holder (110),
and **in that** a periphery of the test material unit (120) matches a shape of the inner surface of the concave housing compartment (112a).

2. The apparatus (100) according to claim 1, wherein the shape of the inner surface of the concave housing compartment (112a) is circular, elliptical, square, rectangular, polygonal, quadrilateral, triangular, parallelogram, pentagonal, hexagonal, heptagonal or octagonal.

3. The apparatus (100) according to claim 1, wherein the holding portion (114) of the holder (110) is an elastic band or wherein the holding portion (114) of the holder (110) is adjustable or stretchable.

4. The apparatus (100) according to claim 1, wherein the holding portion (114) of the holder (110) further comprises a fastening means (114a, 114b).

5. The apparatus (100) according to claim 4, wherein the fastening means (114a, 114b) of the holding portion (114) is a hook-and-loop, a button closure, or a buckle closure.

6. The apparatus (100) according to claim 1, wherein the test material unit (120) is an orthopedic surgery metal selected from the group consisting of aluminum, chromium, cobalt, molybdenum, nickel, vanadium, titanium and Ti6AI-4V.

7. The apparatus (100) according to claim 1, wherein the test material unit (120) is a dental implant material selected from the group consisting of titanium, zirconium, aluminum, and vanadium or a dental amalgam consisting essentially of a liquid mercury and a metal alloy mixture having mercury, silver, tin, copper, or combinations of two or more thereof.

8. The apparatus (100) according to claim 1, wherein the top surface (121) is smaller than the bottom surface (122) and the top surface (121) is fitted and secured in the concave housing compartment (112a) of the holder (110) by one or more fastening means.

9. The apparatus according to claim 1, wherein the test material unit (120) is an orthopedic surgery metal alloy.

10. The apparatus according to claim 1, wherein the test material unit (120) is an orthopedic surgery metal alloy of chromium, cobalt, or molybdenum.

11. The apparatus of claim 1, wherein the implant material is the metal alloy.

12. A method for contacting a test material with a skin of a subject, prior to or after the subject undergoing implant surgery, wherein an implant material is used as the test material to test tissue sensitivity of the subject to the implant material, comprising:
applying the apparatus according to claim 1 on the skin of the subject, such that the test material unit (120) of the apparatus makes the direct contact with the skin; and
determining a presence of skin reactivity based on one or more cutaneous reactions of the skin having the direct contact with the test material unit (120) of the apparatus.

13. The method of claim 12, wherein the implant material is an orthopedic surgery metal selected from the group consisting of aluminum, chromium, cobalt, molybdenum, nickel, vanadium, titanium, and Ti6AI-4V.

## Patentansprüche

1. Vorrichtung (100) zum In-Kontakt-Bringen eines Testmaterials mit der Haut einer Testperson, umfassend:
einen Halter (110) und
eine Testmaterialeinheit (120),
wobei der Halter (110) ferner einen Testabschnitt (112) und einen Halteabschnitt (114) umfasst,
wobei der Halter (110) aus einem inerten und hypoallergenen Material gebildet ist;
wobei der Testabschnitt (112) eine konkave Gehäusekammer (112a) mit einer Öffnung umfasst, die der Haut der Testperson zugewandt ist, wenn die Vorrichtung getragen wird,
wobei der Halteabschnitt so konfiguriert ist, dass er bewirkt, dass eine Bodenfläche (122) der Testmaterialeinheit (120) in direkten Kontakt mit der Haut einer Testperson kommt;
wobei die Testmaterialeinheit (120) aus einem Implantatmetall oder einer Metalllegierung besteht, wobei die konkave Gehäusekammer (112a) so angepasst ist, dass sie eine obere Oberfläche (121) der Testmaterialeinheit (120) aufnimmt und,
wobei der Halteabschnitt (114) des Halters (110) ausgewählt ist aus einer Gruppe bestehend aus einem Armkettchen, einem Band, einem Armband, einem Brustband, einem Beinband, einem Fußkettchen, einem Gürtel, einem Taillengürtel und einem uhrarmbandartigen Halter,
**dadurch gekennzeichnet, dass**
der Testabschnitt (112) ferner ein geschlossenes, von der Haut abgewandtes Ende aufweist,
die konkave Gehäusekammer (112a) so beschaffen ist, dass sie die Testmaterialeinheit (120) darin aufnimmt und berührt,
die Testmaterialeinheit (120) an der konkaven Gehäusekammer (112a) des Testabschnitts (112) des Halters (110) angebracht werden kann,
und dass eine Peripherie der Testmaterialeinheit (120) einer Form der Innenfläche der konkaven Gehäusekammer (112a) entspricht.

2. Vorrichtung (100) nach Anspruch 1, wobei die Form der Innenfläche der konkaven Gehäusekammer (112a) kreisförmig, elliptisch, quadratisch, rechteckig, polygonal, viereckig, dreieckig, parallelogrammförmig, fünfeckig, sechseckig, siebeneckig oder achteckig ist.

3. Vorrichtung (100) nach Anspruch 1, wobei der Halteabschnitt (114) des Halters (110) ein elastisches Band ist oder wobei der Halteabschnitt (114) des Halters (110) einstellbar oder dehnbar ist.

4. Vorrichtung (100) nach Anspruch 1, wobei der Halteabschnitt (114) des Halters (110) ferner ein Befestigungsmittel (114a, 114b) umfasst.

5. Vorrichtung (100) nach Anspruch 4, wobei das Befestigungsmittel (114a, 114b) des Halteabschnitts (114) ein Klettverschluss, ein Knopfverschluss oder ein Schnallenverschluss ist.

6. Vorrichtung (100) nach Anspruch 1, wobei die Testmaterialeinheit (120) ein orthopädisches chirurgisches Metall ist, das ausgewählt ist aus der Gruppe bestehend aus Aluminium, Chrom, Kobalt, Molybdän, Nickel, Vanadium, Titan und Ti6AI-4V.

7. Vorrichtung (100) nach Anspruch 1, wobei die Testmaterialeinheit (120) ein Zahnimplantatmaterial ist, das ausgewählt ist aus der Gruppe bestehend aus Titan, Zirkonium, Aluminium und Vanadium oder einem Dentalamalgam, das im Wesentlichen aus einem flüssigen Quecksilber und einer Metalllegierungsmischung mit Quecksilber, Silber, Zinn, Kupfer oder Kombinationen von zwei oder mehr davon besteht.

8. Vorrichtung (100) nach Anspruch 1, wobei die obere Oberfläche (121) kleiner als die untere Oberfläche (122) ist und die obere Oberfläche (121) in der konkaven Gehäusekammer (112a) des Halters (110) durch ein oder mehrere Befestigungsmittel eingepasst und befestigt ist.

9. Vorrichtung nach Anspruch 1, wobei die Testmaterialeinheit (120) eine orthopädische chirurgische Metalllegierung ist.

10. Vorrichtung nach Anspruch 1, wobei die Testmaterialeinheit (120) eine orthopädische chirurgische Metalllegierung aus Chrom, Kobalt oder Molybdän ist.

11. Vorrichtung nach Anspruch 1, wobei das Implantatmaterial eine Metalllegierung ist.

12. Verfahren zum In-Kontakt-Bringen eines Testmaterials mit der Haut einer Testperson, bevor oder nachdem sich die Testperson einer Implantationschirurgie unterzieht, wobei ein Implantatmaterial als das Testmaterial verwendet wird, um die Gewebeempfindlichkeit der Testperson gegenüber dem Implantatmaterial zu testen, umfassend:
Anlegen der Vorrichtung nach Anspruch 1 an die Haut der Testperson, so dass die Testmaterialeinheit (120) der Vorrichtung den direkten Kontakt mit der Haut herstellt; und
Bestimmung des Vorhandenseins einer Hautreaktivität auf der Grundlage einer oder mehrerer Hautreaktionen der Haut, die in direktem Kontakt mit der Testmaterialeinheit (120) der Vorrichtung steht.

13. Verfahren nach Anspruch 12, wobei das Implantatmaterial ein orthopädisches chirurgisches Metall ist, das ausgewählt ist aus der Gruppe bestehend aus Aluminium, Chrom, Kobalt, Molybdän, Nickel, Vanadium, Titan und Ti6AI-4V.

## Revendications

1. Appareil (100) pour mettre en contact un matériau d'essai avec la peau d'un sujet, comprenant:
un support (110) et
une unité de matériel d'essai (120),
dans lequel le support (110) comprend en outre une partie de test (112) et une partie de maintien (114),
dans lequel le support (110) est constitué d'un matériau inerte et hypoallergénique;
dans lequel la partie test (112) comprend un compartiment concave (112a) dont l'ouverture est orientée vers la peau du sujet lorsque l'appareil est porté,
dans lequel la partie de maintien est configurée pour que la surface inférieure (122) de l'unité de matériau de test (120) soit en contact direct avec la peau d'un sujet;
dans lequel l'unité de matériau d'essai (120) est constituée d'un métal d'implant ou d'un alliage métallique, dans lequel le compartiment concave du boîtier (112a) est adapté pour recevoir une surface supérieure (121) de l'unité de matériau d'essai (120) et,
dans lequel la partie de maintien (114) du support (110) est choisie dans le groupe constitué d'un bracelet, d'une bande, d'un bracelet de poignet, d'un bracelet de poitrine, d'un bracelet de jambe, d'un bracelet de cheville, d'une ceinture, d'une ceinture de taille et d'un support de type bracelet-montre,
**caractérisé par le fait que**
la partie test (112) comprend en outre une extrémité fermée tournée vers l'extérieur de la peau,
le compartiment concave du boîtier (112a) est adapté pour recevoir et mettre en contact l'unité de matériau d'essai (120) qui s'y trouve,
l'unité de matériau d'essai (120) peut être montée sur le compartiment concave (112a) de la partie d'essai (112) du support (110),
et en ce qu'une périphérie de l'unité de matériau d'essai (120) correspond à une forme de la surface intérieure du compartiment concave du boîtier (112a).

2. Appareil (100) selon la revendication 1, dans lequel la forme de la surface intérieure du compartiment concave (112a) est circulaire, elliptique, carrée, rectangulaire, polygonale, quadrilatérale, triangulaire, parallélogramme, pentagonale, hexagonale, heptagonale ou octogonale.

3. Appareil (100) selon la revendication 1, dans lequel la partie de maintien (114) du support (110) est une bande élastique ou dans lequel la partie de maintien (114) du support (110) est ajustable ou extensible.

4. Appareil (100) selon la revendication 1, dans lequel la partie de maintien (114) du support (110) comprend en outre un moyen de fixation (114a, 114b).

5. Appareil (100) selon la revendication 4, dans lequel le moyen de fixation (114a, 114b) de la partie de maintien (114) est un crochet et une boucle, une fermeture à bouton ou une fermeture à boucle.

6. Appareil (100) selon la revendication 1, dans lequel l'unité de matériau d'essai (120) est un métal de chirurgie orthopédique choisi dans le groupe constitué par l'aluminium, le chrome, le cobalt, le molybdène, le nickel, le vanadium, le titane et le Ti6AI-4V.

7. Appareil (100) selon la revendication 1, dans lequel l'unité de matériau d'essai (120) est un matériau d'implant dentaire choisi dans le groupe constitué par le titane, le zirconium, l'aluminium et le vanadium ou un amalgame dentaire constitué essentiellement d'un mercure liquide et d'un mélange d'alliages métalliques contenant du mercure, de l'argent, de l'étain, du cuivre ou des combinaisons de deux ou plusieurs de ces éléments.

8. Appareil (100) selon la revendication 1, dans lequel la surface supérieure (121) est plus petite que la surface inférieure (122) et la surface supérieure (121) est montée et fixée dans le compartiment concave (112a) du support (110) par un ou plusieurs moyens de fixation.

9. Appareil selon la revendication 1, dans lequel l'unité de matériau d'essai (120) est un alliage métallique de chirurgie orthopédique.

10. Appareil selon la revendication 1, dans lequel l'unité de matériau d'essai (120) est un alliage métallique de chrome, de cobalt ou de molybdène pour la chirurgie orthopédique.

11. Appareil de la revendication 1, dans lequel le matériau de l'implant est l'alliage métallique.

12. Méthode pur mettre en contact un matériau d'essai avec la peau d'un sujet, avant ou après que le sujet ait subi une chirurgie d'implantation, dans laquelle un matériau d'implantation est utilisé comme matériau d'essai pour tester la sensibilité des tissus du sujet au matériau d'implantation, comprenant:
appliquer l'appareil selon la revendication 1 sur la peau du sujet, de telle sorte que l'unité de matériau d'essai (120) de l'appareil entre en contact direct avec la peau; et
déterminer la présence d'une réactivité cutanée sur la base d'une ou plusieurs réactions cutanées de la peau en contact direct avec l'unité de matériau d'essai (120) de l'appareil.

13. Méthode de la revendication 12, dans laquelle le matériau de l'implant est un métal de chirurgie orthopédique choisi dans le groupe constitué par l'aluminium, le chrome, le cobalt, le molybdène, le nickel, le vanadium, le titane et le Ti6AI-4V.
